# EUROPEAN PATENT SPECIFICATION

(11) **EP 3 995 143 B1**
(45) Date of publication and mention of the grant of the patent: **05.11.2025**
(21) Application number: 20834986.0
(22) Date of filing: 26.06.2020
(51) Int. Cl.: A61K 35/583, A61P 29/00, A61P 19/02, A23L 33/10, A61K 8/98, A61Q 19/08

(54) **COMPOSITION FOR PREVENTING OR TREATING RHEUMATOID ARTHRITIS, COMPRISING SNAKE VENOM**
SCHLANGENGIFTHALTIGE ZUSAMMENSETZUNG ZUR VORBEUGUNG ODER BEHANDLUNG VON RHEUMATOIDER ARTHRITIS
COMPOSITION POUR PRÉVENIR OU TRAITER LA POLYARTHRITE RHUMATOÏDE, COMPRENANT DU VENIN DE SERPENT

(30) Priority: 02.07.2019 KR 20190079560; 02.07.2019 KR 20190079559
(43) Date of publication of application: 11.05.2022
(73) Proprietor: AJOU UNIVERSITY INDUSTRY-ACADEMIC COOPERATION FOUNDATION, Gyeonggi-cho, 16499 (KR)
(72) Inventor: SOHN, Seong Hyang, Suwon-si Gyeonggi-do 16699 (KR)
(74) Representative: Roos, Peter
(86) International application number: PCT/KR2020/008413
(87) International publication number: WO 2021/002642

(56) References cited:
- CN-A- 109 758 480
- KR-B1- 101 802 515
- KR-B1- 101 802 515
- US-A- 4 341 762
- US-A- 4 341 762
- US-A1- 2008 279 958
- US-A1- 2009 209 468
- US-A1- 2009 209 468
- BURBRINK BURBRINK FRANK FRANK T. T. ET AL: "Considering gene flow when using coalescent methods to delimit lineages of North American pitvipers of the genus Agkistrodon", ZOOLOGICAL JOURNAL OF THE LINNEAN SOCIETY, 1 February 2015 (2015-02-01), Oxford, pages 505 - 526, XP093051136, Retrieved from the Internet <URL:https://onlinelibrary.wiley.com/doi/full/10.1111/zoj.12211> [retrieved on 20230601], DOI: 10.1111/zoj.12211
- BURBRINK BURBRINK FRANK FRANK T. T. ET AL: "Considering gene flow when using coalescent methods to delimit lineages of North American pitvipers of the genus Agkistrodon", ZOOLOGICAL JOURNAL OF THE LINNEAN SOCIETY, 1 February 2015 (2015-02-01), Oxford, pages 505 - 526, XP93051136, Retrieved from the Internet <URL:https://academic.oup.com/zoolinnean/article/173/2/505/2449780> [retrieved on 20230601], DOI: 10.1111/zoj.12211

## Description

### [Technical Field]

The present invention relates to a composition for preventing or treating rheumatoid arthritis, comprising snake venom derived from *Naja melanoleuca,* more specifically the present invention relates to a pharmaceutical composition for preventing or treating rheumatoid arthritis, comprising snake venom derived from *Naja melanoleuca,* which is a type of snake and a food composition thereof for use in treating rheumatoid arthritis

### [Background Art]

In general, arthritis refers to a disease that causes inflammation in the damaged area after the joint is damaged, resulting in the joint area not recovering, and is known to cause serious pain while inflammation occurs at the damaged area. Although such arthritis is considered as a type of inflammatory disease due to inflammation, which is the final symptom, inflammation is a kind of natural in vivo response accompanied by joint damage in vivo, and because the inflammation is not the cause of arthritis, generally arthritis is classified into osteoarthritis, which is a type of degenerative disease, and rheumatoid arthritis, which is a type of immune disease, due to the cause of joint damage.

Among these, osteoarthritis is defined as a chronic degenerative disease accompanied by inflammation and pain which are caused by gradual damage of the articular cartilage due to degeneration in the extracellular matrix constituting the articular cartilage, and the prevalence of osteoarthritis is about 40% in people aged 50 years or older, and is higher in women than in men as the age increases. Osteoarthritis is caused by a variety of reasons including degenerative changes, immune system abnormalities, infections, traumas and metabolic disorders, etc., and the factors associated with occurrence of osteoarthritis are known as nitric oxide (NO), cytokines and proteolytic enzymes, etc.

In addition, rheumatoid arthritis is a type of representative systemic chronic autoimmune diseases that mainly invade joints, and it is known that genetic-environmental factors act in its pathogenesis, and genetic factors contribute about 60%. It is known that rheumatoid arthritis causes irreversible joint deformation and disability over time if its disease activity is not controlled, and joint destruction is affected by the degree of drug response to immunosuppressive agents and biological agents, etc. for the control.

As such, unlike osteoarthritis which occurs naturally due to increasing age, it is impossible to predict the occurrence time of rheumatoid arthritis, and because it is a type of natural autoimmune diseases, it is impossible to completely cure them, and therefore, many studies are being conducted in the direction of alleviating symptoms rather than treating them, wherein for the purpose of alleviating pain caused by inflammation, research to mainly develop the anti-inflammatory agents is being actively conducted. For example, non-steroidal anti-inflammatory agents, a type of anti-inflammatory agent, were developed for the purpose of alleviating the symptoms of rheumatoid arthritis, but it has been reported that these non-steroidal anti-inflammatory agents cause side effects in various organs in the body such as the gastrointestinal tract, kidney, heart, liver, etc. so that the development of more effective therapeutic agents is required.

On the other hand, snake venom refers to a mixture of various toxins secreted by poison glands, which are special salivary glands of poisonous snakes, and is known to contain various proteolytic enzymes including proteases and various polypeptides that show toxic to cells. It is known that the snake venom is relatively strong against oxidation, and even if detoxified, its recovery is reversible, but it is very weak against reduction and thus is simply detoxified by cysteine. Recently, the pharmacological activity of the snake venom has been investigated, and accordingly, the development as therapeutic agents for treating various diseases is accelerating.

For example, Korean Patent Application Laid-Open No. 10-2009-0036908 discloses a composition for preventing or treating arteriosclerosis, comprising saxatilin which is disintegrin derived from snake venom, Korean Patent Application Laid-Open No. 10-2016-0118417 discloses a composition for alleviating or treating atopic skin diseases, comprising snake venom of mamushi, and Japanese Patent Application Laid-Open No. 2010-075194 discloses a formulation for treating inflammatory response-related diseases, comprising snake venom-containing complex as an active ingredient. Korean Patent No. 10-1802515 discloses a pharmaceutical composition for preventing or treating inflammatory diseases, comprising snake venom derived from AP(*Agkistrodon piscivorus piscivorus*) as an active ingredient, Korean Patent No. 10-1802514 discloses a pharmaceutical composition for preventing or treating inflammatory diseases, comprising snake venom derived from *NM*(*Naja melanoleuca*) as an active ingredient. US 2009/209468 A1 relates to methods and compositions for treating arthritic conditions such as osteoporosis and rheumatoid arthritis US4341762A discloses snake venom from *Agkistrodon piscivorus* for treating rheumatoid arthritis in mammals.

Under this background, the inventors have made extensive research efforts to develop a formulation that can effectively treat rheumatoid arthritis and, as a result, confirm that snake venom derived *Naja melanoleuca* can improve the symptoms of rheumatoid arthritis, a type of autoimmune diseases, so that the present invention was completed.

### [Detailed Description]

### [Technical Problem]

The main object of the present invention is to provide a pharmaceutical composition for preventing or treating rheumatoid arthritis, comprising snake venom derived from *Naja melanoleuca.*

Another object of the present invention is to provide a quasi-drug composition for preventing or improving rheumatoid arthritis, comprising snake venom derived from *Naja melanoleuca.*

Another object of the present invention is to provide a food composition for preventing or improving rheumatoid arthritis, comprising snake venom derived from *Naja melanoleuca.*

Another object of the present invention is to provide a cosmetic composition for preventing or improving rheumatoid arthritis, comprising snake venom derived from *Naja melanoleuca.*

Another object of the present invention is to provide a method of treating rheumatoid arthritis using the pharmaceutical composition.

### [Technical Solution]

The present invention is defined in the claims. In particular, the present invention provides a pharmaceutical composition for use in preventing or treating rheumatoid arthritis, comprising snake venom derived from *Naja melanoleuca.*

In the present invention, the term "extract" refers to a result product such as a liquid component obtained by immersing a target material in various solvents and then extracting it for a certain period of time at room temperature or in heated state, and a solid powder obtained by removing the solvent from the liquid component. Moreover, in addition to the result product, it can be comprehensively interpreted as including all of the dilutions of the result product, concentrates thereof, coarse purified products thereof, and purified products thereof. Accordingly, extract of snake venom derived from *Naja melanoleuca* provided in the present invention can be interpreted as including an extract itself and the extract of all formulations that can be formed using the extract, such as the extract obtained by extracting snake venom derived from *Naja melanoleuca,* dilutions or concentrates of the extract, dried product obtained by drying the extract, coarse purified or purified product of the extract; or their mixture, etc.

The method of obtaining the extract of snake venom derived from *Naja melanoleuca* of the present invention is not particularly limited, and can be extracted according to a method commonly used in the art. Non-limiting examples of the extraction method include hot water extraction, ultrasonic extraction, filtration, reflux extraction, etc., and these may be performed alone or in combination of two or more methods.

In the present invention, the type of solvent used for the extraction is not particularly limited, and any solvent known in the art may be used. Non-limiting examples of the extraction solvent include water, alcohol or mixed solvents thereof, etc., and these may be used alone or as a mixture of one or more, and specifically water may be used. When alcohol is used as a solvent, alcohol with 1 to 4 carbon atoms may be specifically used.

In the present invention, the term "fraction" means a result obtained by performing fractionation in order to separate a specific component or a group of specific components from a mixture containing several different components.

The fractionation method of obtaining the fraction in the present invention is not particularly limited, and may be performed according to a method commonly used in the art. Non-limiting examples of the fractionation method include solvent fractionation performed by treating various solvents, ultrafiltration fractionation performed by passing through an ultrafiltration membrane having a constant molecular weight cut-off value, chromatographic fractionation method for performing various chromatography (those prepared for separation according to size, charge, hydrophobicity or affinity), and combinations thereof. Specifically, there is a method of obtaining a fraction from the extract by treating the extract obtained by extracting snake venom derived from *Naja melanoleuca* of the present invention with a predetermined solvent.

In the present invention, the type of the fractionation solvent used to obtain the fraction is not particularly limited, and any solvent known in the art may be used. Non-limiting examples of the fractionation solvent include polar solvents such as water, alcohol with 1 to 4 carbon atoms; non-polar solvents such as hexane, ethyl acetate, chloroform, dichloromethane; or a mixed solvents thereof. These may be used alone or in combination of one or more, but is not limited thereto.

In addition, the extract or fraction may be prepared and used in a dry powder form after extraction, but is not limited thereto.

In the present invention, the term "rheumatoid arthritis" is an inflammatory autoimmune disease that simultaneously affects multiple joints, and means the disease causing joint damage due to chronic arthritis, or invading other organs other than the joints to show symptoms throughout the body.

The autoimmune disease refers to a disease in which an abnormality in immune function occurs so that immune cells responsible for immune function attack organs or tissues of our body to occur the disease.

In the present invention, the term "prevention" refers to any action of inhibiting or delaying rheumatoid arthritis by administering a composition comprising snake venom derived from *Naja melanoleuca.*

In the present invention, the term "treatment" refers to any action of improving or beneficially changing the symptoms of rheumatoid arthritis by administering a composition comprising snake venom derived from *Naja melanoleuca.*

A pharmaceutical composition of the present invention may contain snake venom derived from *Naja melanoleuca* in an amount of 0.001 to 80, specifically 0.001 to 70, more specifically 0.001 to 60% by weight based on the total weight of the composition, but is not limited thereto.

In addition, the pharmaceutical composition may further comprise a pharmaceutically acceptable carrier, excipient or diluent commonly used in the manufacture of a pharmaceutical composition, and the carrier can include a non-naturally occurring carrier. The carrier, excipient and diluent can include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and mineral oil.

In addition, the pharmaceutical composition can be formulated and used in the form of tablet, pill, powder, granule, capsule, suspension, internal solution, emulsion, syrup, sterilized aqueous solution, non-aqueous solvent, suspension, emulsion, freeze-dried formulation, transdermal absorbents, gels, lotions, ointments, creams, patches, cataplasmas, pastes, sprays, skin emulsions, skin suspensions, transdermal delivery patches, drug-containing bandages or suppositories according to a conventional method, respectively. Specifically, it can be prepared using a diluent or excipient such as a filler, a weight agent, a binder, a wetting agent, a disintegrant, and a surfactant commonly used in the case of formulation. Solid preparations for oral administration include tablets, pills, powders, granules, capsules, and the like, but are not limited thereto. Such a solid preparation may be prepared by mixing at least one or more excipients, for example, starch, calcium carbonate, sucrose, lactose, gelatin, and the like. Also, in addition to simple excipients, lubricants such as magnesium stearate and talc may also be used. In addition to liquids and liquid paraffin for oral use, it can be prepared by adding various excipients, for example, wetting agents, sweetening agents, fragrances, preservatives, and the like. Formulations for parenteral administration include sterile aqueous solutions, non-aqueous solutions, suspensions, emulsions, lyophilized formulations and suppositories. Non-aqueous solvents and suspending agents include propylene glycol, polyethylene glycol, vegetable oils such as olive oil, and injectable esters such as ethyl oleate. As the base of the suppository, Witepsol, Macrogol, Tween 61, cacao butter, laurin fat, glycerogelatin, etc. can be used.

Also described herein is a method of treating rheumatoid arthritis, comprising administering the pharmaceutical composition to a subject suspected of rheumatoid arthritis other than humans.

The term "administration" refers to the action of introducing a composition comprising snake venom derived from *Naja melanoleuca* to a subject by an appropriate method.

As used herein, the term "subject" refers to all animals including humans, rats, mice, and livestock that rheumatoid arthritis is developed or can be developed. As a specific example, it may be a mammal including human.

A pharmaceutical composition of the present invention is administered in a pharmaceutically effective amount. As used herein, the term "pharmaceutically effective amount" means an amount sufficient to treat a disease with a reasonable benefit/risk ratio applicable to medical treatment, and the effective dose level depends on factors including the type of the subject and severity, age, sex, activity of the drug, sensitivity to the drug, time of administration, route of administration, and excretion rate, duration of treatment, concomitant drugs, and other factors well known in the medical field. For example, snake venom derived from *Naja melanoleuca* may be administered at a dose of 0.01 to 500 mg/kg per day, specifically 0.1 to 50 mg/kg, and it is administered once a day or can be administered in several divided doses.

The pharmaceutical composition may be administered as individual therapeutic agents or may be administered in combination with other therapeutic agents, and may be administered sequentially or simultaneously with conventional therapeutic agents. And it may be administered single or multiple. Taking all of the above factors into consideration, it is important to administer an amount that can obtain the maximum effect with a minimum amount without side effects, and can be easily determined by those skilled in the art.

In addition, the pharmaceutical composition may be administered orally or parenterally (eg, intravenously, subcutaneously, dermally, intraperitoneally or topically) according to the desired method, and although the dosage may vary depending on the patient's condition, weight, and the degree of the disease, drug form, administration route and time, it may be appropriately selected by those skilled in the art.

Also described herein is a quasi-drug composition for preventing or improving rheumatoid arthritis, comprising snake venom derived from *Naja melanoleuca,* or a fraction thereof.

In this case, the definition of the extract, fraction, rheumatoid arthritis, prevention and improvement is the same as described above.

In the present invention, the term "quasi-drug" means a fiber, a rubber product or similar things used for the purpose of treating, alleviating, curing or preventing diseases of humans or animals, articles that have a weak effect on the human body or do not act directly on the human body and that are not instruments, machines, and similar things, articles that are equivalent to one of the preparations used for sterilization, insecticidal and similar uses for prevention of infection, that are used for the purpose of diagnosing, treating, alleviating, curing or preventing diseases of humans or animals and that are not instruments, machines or devices, and articles that are used for the purpose of pharmacologically affecting the structure and function of humans and animals and that are other than instruments, machines or devices, and also includes external skin preparation and personal hygiene products.

When snake venom derived from *Naja melanoleuca* of the present invention is added to a quasi-drug composition for the purpose of preventing or improving rheumatoid arthritis, snake venom derived from *Naja melanoleuca* is added as it is or it can be used together with other quasi-drug ingredients, and can be used appropriately according to a conventional method. The mixing amount of the active ingredient may be appropriately determined according to the purpose of use (prevention, health or therapeutic care).

The quasi-drug composition is not particularly limited thereto, but includes personal hygiene products, external skin preparation, disinfectant cleaner, shower foam, wet tissue, detergent soap, hand wash, mask or ointment. The external skin preparation is not particularly limited thereto, but specifically may be prepared and used in the form of an ointment, lotion, spray, patch, cream, powder, suspension, gel or gel. The personal hygiene products is not particularly limited thereto, and specifically, may be soap, wet tissue, tissue, shampoo, toothpaste, hair care product, air freshener gel or cleaning gel.

Another embodiment of the present invention provides a food composition for use in preventing or improving rheumatoid arthritis, comprising snake venom derived from *Naja melanoleuca.*

In this case, the definition of the extract, fraction, rheumatoid arthritis, prevention and improvement is the same as described above.

In the present invention, the term "improvement" refers to any action that at least decrease a parameter, related to a condition to be treated by administration of a composition containing the extract, for example, the degree of symptoms.

As In the present invention, the term "food" is meat, sausage, bread, chocolate, candies, snacks, confectionery, pizzas, ramens, other noodles, gums, dairy products including ice cream, various soups, beverages, tea, drinks, alcohol beverages, vitamin complexes, health functional foods and health foods, etc. and includes all foods in a normal sense.

Since snake venom derived from *Naja melanoleuca,* an extract thereof or a fraction thereof does not induce side effects in vivo even when orally administered, if it is ingested, it can be used very usefully for health promotion purposes because it can be expected to prevent or improve a high level of rheumatoid arthritis.

The health functional food is the same term as food for special health use (FoSHU), and in addition to nutritional supply, it means food with high medical, pharmaceutical effects, processed to efficiently exhibit bioregulatory functions. Here, 'functional' refers to regulating nutrients on the structure and function of the human body or obtaining useful effects for hygiene purposes, such as physiological effects. The food of the present invention can be prepared by a method commonly used in the art, and when prepared, it can be prepared by adding raw materials and ingredients commonly added in the art. In addition, the formulation of the food can be prepared without limitation as long as it is a formulation recognized as a food.

A food composition of the present invention can be prepared in various formulations, and unlike general medicines, since it is made from natural substances as a raw material, there is an advantage in that there are no side effects that may occur during long-term use of the drug, and due to excellent portability, the food of the present invention can be ingested as an adjuvant to enhance the prevention or improvement effect of rheumatoid arthritis.

The health food means food having an active health maintenance or promotion effect compared to general food, and health supplement food means food for the purpose of health supplementation. In some cases, the terms health functional food, health food, and health supplement are used interchangeably.

Specifically, the health functional food is the food prepared that snake venom derived from *Naja melanoleuca* of the present invention, an extract thereof or a fraction thereof is added to food materials such as beverages, teas, spices, gums, confectionery, or made by encapsulation, pulverization, suspension, etc., and when the health functional food is ingested, certain health effects are obtained, and unlike general medicines, since foods is used as a raw material, there is an advantage in that there are no side effects that may occur during long-term use of the drug.

The food composition may further contain a physiologically acceptable carrier, wherein the type of carrier is not particularly limited, and any carrier commonly used in the art may be used.

In addition, the food composition may contain an additional ingredient that is commonly used in a food composition to improve odor, taste, vision, and the like. For example, vitamins A, C, D, E, B1, B2, B6, B12, niacin, biotin, folate, pantothenic acid and the like may be included. In addition, minerals such as zinc (Zn), iron (Fe), calcium (Ca), chromium (Cr), magnesium (Mg), manganese (Mn), copper (Cu), chromium (Cr); and amino acids such as lysine, tryptophan, cysteine, valine and the like may be included.

In addition, the food composition may contain food additives, including preservatives (potassium sorbate, sodium benzoate, salicylic acid, sodium dehydroacetate, etc.), disinfectants (bleaching powder and high bleaching powder, sodium hypochlorite, etc.), antioxidants (butylhydroxyanisole (BHA), butylhydroxytoluene (BHT), etc.), coloring agents (tar pigments, etc.), coloring agents (sodium nitrite, etc.), bleach (sodium sulfite), seasonings (MSG sodium glutamate, etc.), sweeteners (dulcin, cyclamate, saccharin, sodium, etc.), flavorings (vanillin, lactones, etc.), swelling agents (alum, D-potassium hydrogen tartrate, etc.), strengthening agents, emulsifiers, thickening agents (flavors), film agents, gum base agents, foam inhibitors, solutions, improving agents, etc. The additives may be selected according to the type of food and used in an appropriate amount.

As an example of a food composition of the present invention, a health beverage composition may be used, and in this case, it may contain, like conventional beverages, various flavoring agents or natural carbohydrates as additional ingredients. The above-mentioned natural carbohydrates may be monosaccharides such as glucose and fructose; disaccharides such as maltose and sucrose; polysaccharides such as dextrin and cyclodextrin; a sugar alcohol such as xylitol, sorbitol, and erythritol. Sweetening agents, including natural sweetening agents such as taumatine and stevia extract; synthetic sweetening agents such as saccharin or aspartame may be used. The ratio of the natural carbohydrates may be generally about 0.01~0.04 g, specifically about 0.02~0.03 g per 100 ml of the health beverage composition of the present invention.

In addition to the above, the health beverage composition may contain various nutrients, vitamins, electrolytes, flavoring agents, colorants, pectic acid, salts of pectic acid, alginic acid, salts of alginic acid, organic acids, protective colloidal thickeners, pH adjusters, stabilizers, preservatives, glycerin, alcohol or carbonation agents, etc. In addition, it may contain the flesh for the production of natural fruit juice, fruit juice beverage, or vegetable beverage. These components can be used independently or in combination. Although the ratio of these additives is not very important, it is generally selected in the range of 0.01 to 0.1 parts by weight per 100 parts by weight of the health beverage composition of the present invention.

Another embodiment of the present invention provides a cosmetic composition for preventing or improving rheumatoid arthritis, comprising snake venom derived from *Naja melanoleuca.*

In this case, the definition of the extract, fraction, rheumatoid arthritis, prevention and improvement is the same as described above.

Snake venom derived from *Naja melanoleuca* may be included snake venom derived from *Naja melanoleuca* in a ratio of 0.1 wt% to 20 wt% based on the total weight of the composition as in a pharmaceutical composition. Specifically, it is included in a ratio of 0.1 wt% to 5 wt%, more specifically 0.4 to 0.6 wt%, and still more specifically 0.5 wt%, based on the total weight of the composition, but is not limited thereto.

Ingredients comprised in a cosmetic composition of the present invention include ingredients commonly used in a cosmetic composition in addition to snake venom derived from *Naja melanoleuca,* for example, one or more additives selected from the group consisting of water, surfactant, moisturizer, low-grade alcohol, a chelating agent, a disinfectant, an antioxidant, a preservative, a color and a fragrance may be further included.

In addition, the cosmetic composition may be prepared in any conventionally prepared formulation, for example, it may be formulated as solutions, emulsions, suspensions, pastes, creams, lotions, gels, powders, sprays, surfactant-containing cleansing agents, oils, soaps, liquid detergents, bath agents, foundations, makeup base, essence, tonic, foam, pack, softening water, sunscreen cream or sun oil, but is not limited thereto.

When the formulation of the present invention is a solution or emulsion, a solvent, solubilizer or emulsifier, for example, water, ethanol, isopropanol, ethyl carbonate, ethyl acetate, benzyl alcohol, benzyl benzoate, propylene glycol, 1,3-butyl glycol oil, glycerol fatty ester, polyethylene glycol or fatty acid ester of sorbitan is used as a carrier component.

When the formulation of the present invention is a suspension, a liquid diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, or tracanth may be used as a carrier component.

When the formulation of the present invention is paste, cream or gel, animal oil, vegetable oil, wax, paraffin, starch, tracanth, cellulose derivative, polyethylene glycol, silicone, bentonite, silica, talc or zinc oxide, etc. can be used as a carrier component.

When the formulation of the present invention is powder or spray, lactose, talc, silica, aluminum hydroxide, calcium silicate or polyamide powder can be used as a carrier component, and in particular, in the case of a spray, a propellant such as chlorofluorohydrocarbon, propane/butane or dimethyl ether may be additionally contained.

When the formulation of the present invention is surfactant-containing cleansing, as carrier components, aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, aliphatic alcohol, fatty acid glyceride, fatty acid diethanolamide, vegetable oil, lanolin derivative or ethoxylated glycerol fatty acid ester may be used.

In addition, ingredients contained in the cosmetic composition may be comprised in amounts generally used in the field of dermatology.

### [Effects of Invention]

Snake venom derived from *Naja melanoleuca* of the present invention can improve, alleviate, or treat symptoms of rheumatoid arthritis, which is a kind of autoimmune diseases, and thus will be able to be widely utilized in the development of therapeutic agents for various autoimmune diseases including rheumatoid arthritis.

### [Brief Description of Drawings]

Embodiments related to *Agkistrodon piscivorus piscivorus* are not encompassed by the present invention.
Fig. 1 is a photograph showing the damaged joint area of arthritis model mice treated with snake venom sample derived from *Agkistrodon piscivorus piscivorus,* and show the change in symptoms after applying snake venom derived from *Agkistrodon piscivorus piscivorus* to the joint area with arthritis once/day for 2 weeks as external skin preparation, wherein it was confirmed that the group applied with PBS used as a control group did not improve but worsened.
Fig. 2 is a graph showing the change of recovered arthritic index score in arthritis model mice treated with snake venom sample derived from *Agkistrodon piscivorus piscivorus* and MTX(methotrexate).
Fig. 3 is a photograph showing the damaged joint area of arthritis model mice treated with snake venom sample derived from *Naja melanoleuca,* and show the change in symptoms after applying snake venom derived from *Naja melanoleuca* to the joint area with arthritis once/day for 2 weeks as external skin preparation, wherein it was confirmed that the group applied with PBS used as a control group did not improve but worsened.
Fig. 4 is a graph showing the change of recovered arthritic index score in arthritis model mice treated with snake venom sample derived from *Naja melanoleuca* and MTX(methotrexate).
Fig. 5 is a graph showing the results of flow cytometry analysis of CCR1 positive cells on peripheral blood monocytes and lymph nodes of arthritis model mice orally administered with MTX.
Fig. 6 is a graph showing the results of flow cytometry analysis of CCR1 positive cells on peripheral blood monocytes and lymph nodes of arthritis model mice treated with snake venom sample derived from *Agkistrodon piscivorus piscivorus.*
Fig. 7 is a graph showing the results of flow cytometry analysis of CCR1 positive cells on peripheral blood monocytes and lymph nodes of arthritis model mice treated with snake venom sample derived from *Naja melanoleuca.*
Fig. 8a is a graph showing the results of measuring the change in CD4 positive cells according to the dose in normal mice orally administered with snake venom derived from *Agkistrodon piscivorus piscivorus.*
Fig. 8b is a graph showing the results of measuring the change in CD25 positive cells according to the dose in normal mice orally administered with snake venom derived from *Agkistrodon piscivorus piscivorus.*
Fig. 8c is a graph showing the results of measuring the change in Foxp3 positive cells according to the dose in normal mice orally administered with snake venom derived from *Agkistrodon piscivorus piscivorus.*
Fig. 9a is a graph showing the results of measuring the changes in CD4 and CD25 positive cells according to the dose in normal mice orally administered with snake venom derived from *Agkistrodon piscivorus piscivorus.*
Fig. 9b is a graph showing the results of measuring the changes in CD25 and Foxp3 positive cells according to the dose in normal mice orally administered with snake venom derived from *Agkistrodon piscivorus piscivorus.*
Fig. 9c is a graph showing the results of measuring the changes in CD4 and Foxp3 positive cells according to the dose in normal mice orally administered with snake venom derived from *Agkistrodon piscivorus piscivorus.*
Fig. 9d is a graph showing the results of measuring the changes in CD4, CD25 and Foxp3 positive regulatory T cells according to the dose in normal mice orally administered with snake venom derived from *Agkistrodon piscivorus piscivorus.*
Fig. 10a is a graph showing the results of measuring the change in CD4 positive cells according to the dose in normal mice orally administered with snake venom derived from *Naja melanoleuca.*
Fig. 10b is a graph showing the results of measuring the change in CD25 positive cells according to the dose in normal mice orally administered with snake venom derived from *Naja melanoleuca.*
Fig. 10c is a graph showing the results of measuring the change in Foxp3 positive cells according to the dose in normal mice orally administered with snake venom derived from *Naja melanoleuca.*
Fig. 11a is a graph showing the results of measuring the changes in CD4 and CD25 positive cells according to the dose in normal mice orally administered with snake venom derived from *Naja melanoleuca.*
Fig. 11b is a graph showing the results of measuring the changes in CD25 and Foxp3 positive cells according to the dose in normal mice orally administered with snake venom derived from *Naja melanoleuca.*
Fig. 11c is a graph showing the results of measuring the changes in CD4 and Foxp3 positive cells according to the dose in normal mice orally administered with snake venom derived from *Naja melanoleuca.*
Fig. 11d is a graph showing the results of measuring the changes in CD4, CD25 and Foxp3 positive regulatory T cells according to the dose in normal mice orally administered with snake venom derived from *Naja melanoleuca.*
Fig. 12a is a graph showing the results of comparing levels of CRP (C-reactive protein) in plasma in normal mice orally administered or skin-applied with snake venom derived from *Agkistrodon piscivorus piscivorus.*
Fig. 12b is a graph showing the results of levels of comparing AST(aspartate aminotransferase) in plasma in normal mice orally administered or skin-applied with snake venom derived from *Agkistrodon piscivorus piscivorus.*
Fig. 12c is a graph showing the results of comparing ALT(alanine transaminase) levels in plasma in normal mice orally administered or skin-applied with snake venom derived from Agkistrodon piscivorus piscivorus.
Fig. 13a is a graph showing the results of comparing levels of CRP (C-reactive protein) in plasma in normal mice orally administered or skin-applied with snake venom derived from *Naja melanoleuca.*
Fig. 13b is a graph showing the results of levels of comparing AST(aspartate aminotransferase) in plasma in normal mice orally administered or skin-applied with snake venom derived from *Naja melanoleuca.*
Fig. 13c is a graph showing the results of comparing ALT(alanine transaminase) levels in plasma in normal mice orally administered or skin-applied with snake venom derived from *Naja melanoleuca.*
Fig. 14 is a graph showing the results of comparing the level of IL-17 in plasma in normal mice orally administered with snake venom derived from *Agkistrodon piscivorus piscivorus.*
Fig. 15 is a graph showing the results of comparing the level of IL-17 in plasma in normal mice orally administered with snake venom derived from *Naja melanoleuca.*
Fig. 16 is a microphotograph showing the joint tissue of arthritis model mice orally administered with MTX or applied on skin with snake venom derived from *Agkistrodon piscivorus piscivorus.*
Fig. 17 is a microphotograph showing the joint tissue of arthritis model mice orally administered with MTX or applied on skin with snake venom derived from *Naja melanoleuca.*

### [Best Mode for Carrying Out the Invention]

Hereinafter, the present invention will be described in more detail through working examples. However, these working examples are for illustrative purposes of the present invention, and the scope of the present invention is not limited to these working examples. Only the examples relating to snake venom derived from *Naja melanoleuca* fit in the scope of the claims. The other examples are for reference.

### Example 1: Production of arthritis model mice

Type 2 collagen solution (10 mM), an adjuvant (Complete freund's adjuvant) and oil (mineral oil) were mixed at a ratio of 1:1:1 (v/v/v) to obtain a mixture, 50 µℓ of the mixture was injected into 1 cm site of the tail vein at the tail base of 8-week-old ICR mice, and 10 days later, was injected once more repeatly. After injection, mice showing arthritis symptoms at 10 to 15 days elapsed time point were selected and used as rheumatoid arthritis model mice.

On the other hand, in order to distinguish in detail the level of symptoms of the selected rheumatoid arthritis model mice, severity score is given as follows (Table 1), and when severity score was 2 or more, it was determined that rheumatoid arthritis has occurred.

**[Table 1]**

| Severity score and symptom criteria of arthritis model mice | |
|---|---|
| Score | Symptom |
| 0 | Normal condition with no symptom |
| 1 | Condition with minimal swelling or redness induced in any one of the toes, insteps, ankles, etc. |
| 2 | Condition with significant edema and redness induced in at least two of the toes, insteps, and ankles |
| 3 | Condition with inflammation and edema at three joint areas from the toes to the ankles |
| 4 | Condition in which significant edema and swelling are observed throughout the entire area from the toes to the ankles, or in which the feet cannot be used freely due to stiffness |

Meanwhile, snake venom sample derived from *Agkistrodon piscivorus piscivorus* or *Naja melanoleuca* used in the present disclosure was purchased from Sigma-Aldrich and used.

### Example 2: Therapeutic effect of snake venom on the symptoms of arthritis model mice

### Example 2-1: Therapeutic effect of snake venom derived from Agkistrodon piscivorus piscivorus on the symptoms of arthritis model mice

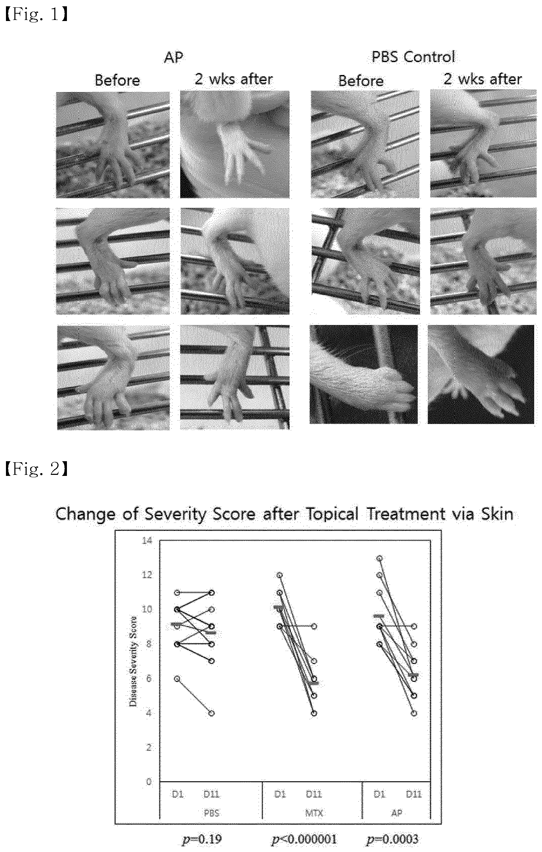

On the skin with the damaged joint area of arthritis model mice produced in Example 1 above, snake venom sample derived from *Agkistrodon piscivorus piscivorus* was applied for 2 weeks, the therapeutic effect of snake venom above was visually verified (Fig. 1). At this time, as a control group, mice applied with PBS were used.

Fig. 1 is a photograph showing the damaged joint area of arthritis model mice treated with snake venom sample derived from *Agkistrodon piscivorus piscivorus.*

In addition, after snake venom sample derived from *Agkistrodon piscivorus piscivorus* was treated at the joint areas of the damaged 4 limbs (one pair of forelimbs and one pair of hind limbs) of arthritis model mice, each severity score was measured (Fig. 2). At this time, as a control group, mice applied with PBS were used, and as a comparative group, mice orally administered with MTX (methotrexate), known as rheumatoid arthritis therapeutic agents, were used.

Fig. 2 is a graph showing the change of recovered arthritic index score in arthritis model mice treated with snake venom sample derived from *Agkistrodon piscivorus piscivorus* and MTX(methotrexate), D1 represents the time point at which 1 day has elapsed after treatment, D11 represents the time point at which 11 days have elapsed after treatment, (∘) represents each arthritis model mouse, (-) is a link of the change between severity score measured at 1 day elapsed time point and severity score measured at 11 days elapsed time point in the same arthritis model mice, and a red mark represents the average value of severity score measured at 1 day and 11 days elapsed time points, respectively.

As shown in Fig. 2, in the case of a control group, severity score measured on days 1 and 11 did not show significant difference, but when MTX was orally administered (comparative group) or snake venom sample derived from *Agkistrodon piscivorus piscivorus* was treated, it was confirmed that the severity score measured on day 11 was significantly decreased compared to the severity score measured on day 1.

Therefore, snake venom sample derived from *Agkistrodon piscivorus piscivorus* was found to have the effect of improving the symptoms of rheumatoid arthritis.

### Example 2-2: Therapeutic effect of snake venom derived from Naja melanoleuca on the symptoms of arthritis model mice

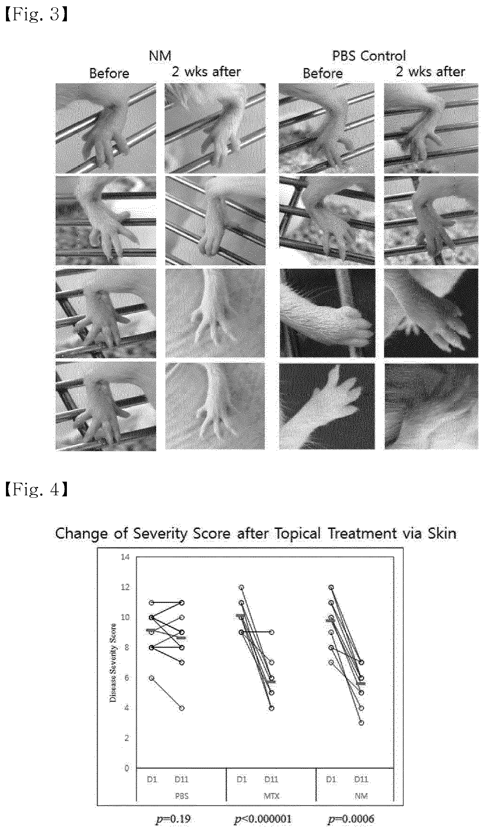

On the skin of the damaged joint area of arthritis model mice produced in Example 1 above, snake venom sample derived from *Naja melanoleuca* was applied for 2 weeks, the therapeutic effect of snake venom above was visually verified (Fig. 3). At this time, as a control group, mice applied with PBS were used.

Fig. 3 is a photograph showing the damaged joint area of arthritis model mice treated with snake venom sample derived from *Naja melanoleuca.*

In addition, after snake venom sample derived from *Naja melanoleuca* was treated at the joint areas of the damaged 4 limbs (one pair of forelimbs and one pair of hind limbs) of arthritis model mice, each severity score was measured (Fig. 4). At this time, as a control group, mice applied with PBS were used, and as a comparative group, mice orally administered with MTX (methotrexate), known as rheumatoid arthritis therapeutic agents, were used.

Fig. 4 is a graph showing the change of recovered arthritic index score in arthritis model mice treated with snake venom sample derived from *Naja melanoleuca* and MTX(methotrexate), D1 represents the time point at which 1 day has elapsed after treatment, D11 represents the time point at which 11 days have elapsed after treatment, (∘) represents each arthritis model mouse, (-) is a link of the change between severity score measured at 1 day elapsed time point and severity score measured at 11 days elapsed time point in the same arthritis model mice, and a red mark represents the average value of severity score measured at 1 day and 11 days elapsed time points, respectively.

As shown in Fig. 4, in the case of a control group, severity score measured on days 1 and 11 did not show significant difference, but when MTX was orally administered (comparative group) or snake venom sample derived from *Naja melanoleuca* was treated, it was confirmed that the severity score measured on day 11 was significantly decreased compared to the severity score measured on day 1.

Therefore, snake venom sample derived from *Naja melanoleuca* was found to have the effect of improving the symptoms of rheumatoid arthritis.

### Example 3: Flow cytometry analysis in arthritis model mice

### Example 3-1: Change in CCR1 positive cells in arthritis model mice orally administered with methotrexate

Peripheral blood monocytes(PBMC) and lymph nodes(LN) were obtained from arthritis model mice orally administered with MTX, and then flow cytometry analysis was performed on these targets.

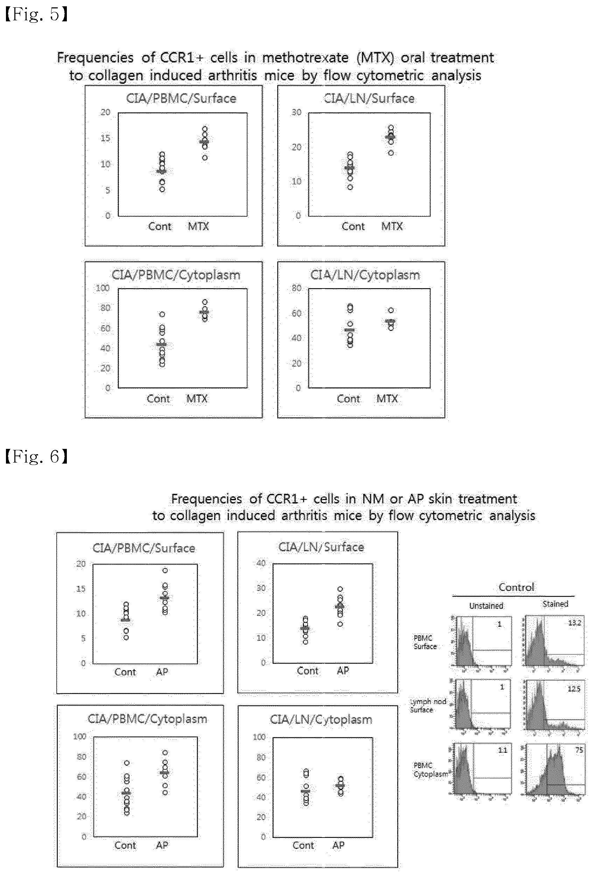

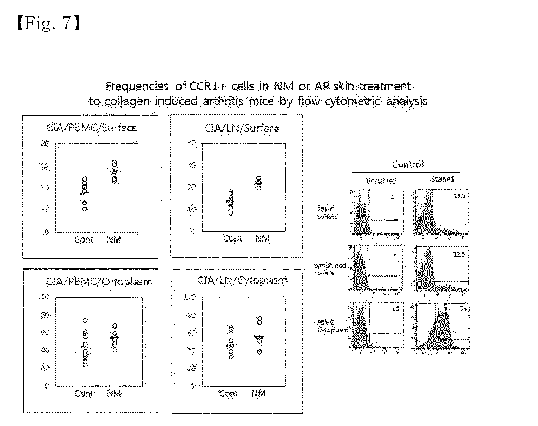

Roughly, an anti-mouse CCR1 antibody was added to cells isolated from peripheral blood monocytes and lymph nodes, and then reacted at 4°C for 30 minutes. Then, in order to perform flow cytometry analysis, BD cantoII flow cytometer was used (Fig. 5). At this time, as a control group, mice administered with PBS were used.

Fig. 5 is a graph showing the results of flow cytometry analysis of CCR1 positive cells on peripheral blood monocytes and lymph nodes of arthritis model mice orally administered with MTX(methotrexate).

As shown in Fig. 5, it was found that the level of CCR1 positive cells in the cell surface and cytoplasm of peripheral blood monocytes and lymph nodes was increased in arthritis model mice orally administered with MTX, known as rheumatoid arthritis therapeutic agents.

From the result of increasing the level of the CCR1 positive cells, it was found that the symptoms of inflammation were improved.

### Example 3-2: Change in CCR1 positive cells in arthritis model mice treated on skin with snake venom derived from Agkistrodon piscivorus piscivorus or Naja melanoleuca

On the skin of the damaged joint area of arthritis model mice produced in Example 1 above, snake venom sample derived from *Agkistrodon piscivorus piscivorus* or *Naja melanoleuca* was applied on skin for 2 weeks, and peripheral blood monocytes(PBMC) and lymph nodes(LN) were obtained from the mice, and then flow cytometry analysis by the method of Example 3-1 above was performed on these targets (Figs. 6 and 7). At this time, as a control group, mice applied with PBS were used.

Fig. 6 is a graph showing the results of flow cytometry analysis of CCR1 positive cells on peripheral blood monocytes and lymph nodes of arthritis model mice treated with snake venom sample derived from *Agkistrodon piscivorus piscivorus.*

Fig. 7 is a graph showing the results of flow cytometry analysis of CCR1 positive cells on peripheral blood monocytes and lymph nodes of arthritis model mice treated with snake venom sample derived from *Naja melanoleuca.*

As shown in Figs. 6 and 7, in the arthritis model mice treated with snake venom of the present invention, it was confirmed that the level of CCR1 positive cells was increased in peripheral blood monocytes and lymph nodes.

These results are similar to the results of oral administration of MTX, known as rheumatoid arthritis therapeutic agents, shown in Fig. 5, and compared with the results of Fig. 5 above, it was found that snake venom of the present invention shows therapeutic effect on rheumatoid arthritis.

### Example 4: Flow cytometry analysis in normal mice

### Example 4-1: Change in CD4, CD25 and Foxp3 positive cell in normal mice orally administered with snake venom derived from Agkistrodon piscivorus piscivorus

It is to confirm whether snake venom derived from *Agkistrodon piscivorus piscivorus* can increase the frequency of regulatory T cells.

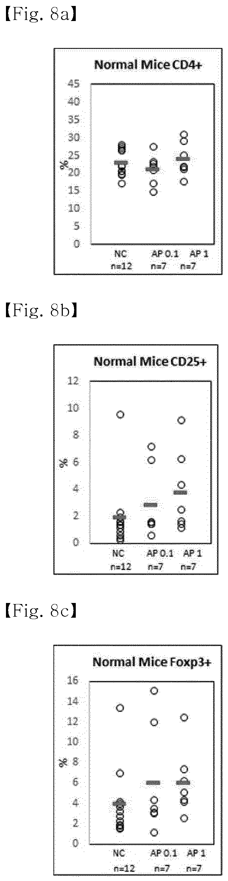

For this, 0.1 µg or 1 µg of snake venom sample derived from *Agkistrodon piscivorus piscivorus* was orally administered to normal mice, and the levels of CD4 positive cells, CD25 positive cells and Foxp3 positive cells from these were confirmed through flow cytometry analysis (Figs. 8a to 8c). At this time, as a control group, mice orally administered with PBS were used. In addition, CD4 positive cells and CD25 positive cells were confirmed by the method of Example 3-1. Finally, Foxp3 positive cells were confirmed through intranuclear staining, and roughly, the cells to be stained were washed twice with 1x permeabilization buffer and then fixed with Fix/perm buffer, and anti-mouse Foxp3 antibody was added and then reacted at 4°C for 30 minutes.

Fig. 8a is a graph showing the results of measuring the change in CD4 positive cells according to the dose in normal mice orally administered with snake venom derived from *Agkistrodon piscivorus piscivorus,* Fig. 8b is a graph showing the results of measuring the change in CD25 positive cells according to the dose in normal mice orally administered with snake venom derived from *Agkistrodon piscivorus piscivorus,* and Fig. 8c is a graph showing the results of measuring the change in Foxp3 positive cells according to the dose in normal mice orally administered with snake venom derived from *Agkistrodon piscivorus piscivorus.*

As shown in Figs. 8a to 8c, among CD4 positive, CD25 positive and Foxp3 positive cells known as markers of regulatory T cells, it was confirmed that the frequency of CD25 positive and Foxp3 positive cells was increased by oral administration of snake venom sample derived from *Agkistrodon piscivorus piscivorus.*

Therefore, since oral administration of snake venom sample derived from *Agkistrodon piscivorus piscivorus* individually increased the frequency of CD25 positive and Foxp3 positive cells, which are the main characteristics of regulatory T cells, from this, it was analyzed that oral administration of snake venom sample derived from *Agkistrodon piscivorus piscivorus* would increase the level of regulatory T cells.

### Example 4-2: Change in regulatory T cells in normal mice orally administered with snake venom derived from Agkistrodon piscivorus piscivorus

Since the results of Example 4-1 above provided a clue that oral administration of snake venom sample derived from *Agkistrodon piscivorus piscivorus* would increase the level of regulatory T cells, in practice, it was to confirm whether oral administration of snake venom sample derived from *Agkistrodon piscivorus piscivorus* could increase the level of regulatory T cells.

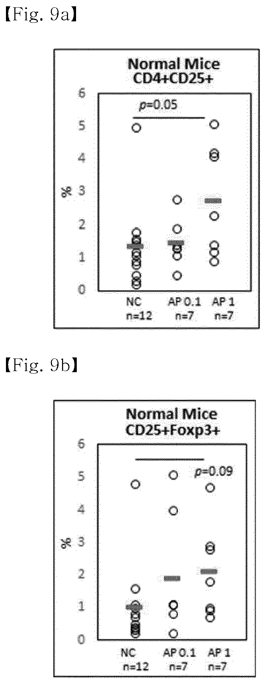

For this, 0.1 µg or 1 µg of snake venom sample derived from *Agkistrodon piscivorus piscivorus* was orally administered to normal mice, and the levels of cells comprising CD4 positive, CD25 positive and Foxp3 positive in combination from these were confirmed through flow cytometry analysis (Figs. 9a to 9d). At this time, as a control group, mice orally administered with PBS were used.

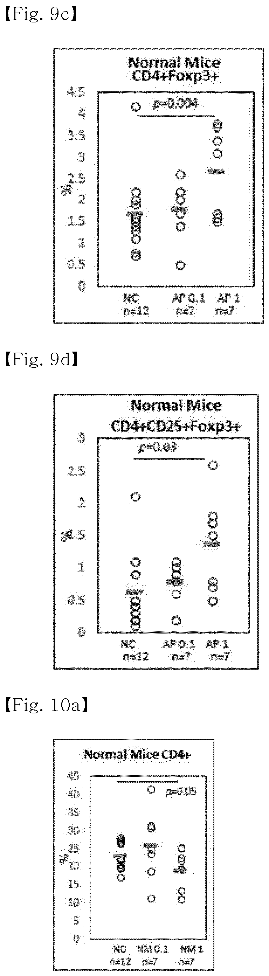

Fig. 9a is a graph showing the results of measuring the changes in CD4 and CD25 positive cells according to the dose in normal mice orally administered with snake venom derived from *Agkistrodon piscivorus piscivorus,* Fig. 9b is a graph showing the results of measuring the changes in CD25 and Foxp3 positive cells according to the dose in normal mice orally administered with snake venom derived from *Agkistrodon piscivorus piscivorus,* Fig. 9c is a graph showing the results of measuring the changes in CD4 and Foxp3 positive cells according to the dose in normal mice orally administered with snake venom derived from *Agkistrodon piscivorus piscivorus,* and Fig. 9d is a graph showing the results of measuring the changes in CD4, CD25 and Foxp3 positive regulatory T cells according to the dose in normal mice orally administered with snake venom derived from *Agkistrodon piscivorus piscivorus.*

As shown in Figs. 9a to 9d, it was confirmed that snake venom sample derived from *Agkistrodon piscivorus piscivorus* increased all of the level of each combination of CD4 positive, CD25 positive and Foxp3 positive, known as the main characteristics of regulatory T cells.

Therefore, it was found that oral administration of snake venom sample derived from *Agkistrodon piscivorus piscivorus* can increase the level of regulatory T cells and, due to the increase in the level of these regulatory T cells, can improve the inflammatory symptoms of rheumatoid arthritis.

### Example 4-3: Changes in CD4, CD25 and Foxp3 positive cells in normal mice orally administered with snake venom derived from Naja melanoleuca

It is to confirm whether snake venom derived from *Naja melanoleuca* can increase the frequency of regulatory T cells.

For this, 0.1 µg or 1 µg of snake venom sample derived from *Naja melanoleuca* was orally administered to normal mice, and the levels of CD4 positive cells, CD25 positive cells and Foxp3 positive cells from these were confirmed through flow cytometry analysis (Figs. 10a to 10c). At this time, as a control group, mice orally administered with PBS were used. In addition, CD4 positive cells and CD25 positive cells were confirmed by the method of Example 3-1. Finally, Foxp3 positive cells were confirmed through intranuclear staining, and roughly, the cells to be stained were washed twice with 1x permeabilization buffer and then fixed with Fix/perm buffer, and anti-mouse Foxp3 antibody was added and then reacted at 4°C for 30 minutes.

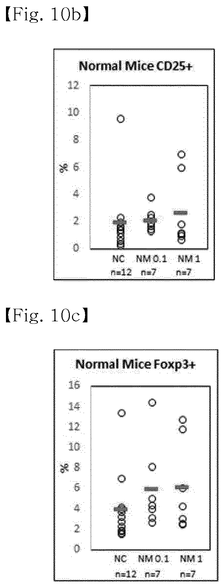

Fig. 10a is a graph showing the results of measuring the change in CD4 positive cells according to the dose in normal mice orally administered with snake venom derived from *Naja melanoleuca,* Fig. 10b is a graph showing the results of measuring the change in CD25 positive cells according to the dose in normal mice orally administered with snake venom derived from *Naja melanoleuca,* and Fig. 10c is a graph showing the results of measuring the change in Foxp3 positive cells according to the dose in normal mice orally administered with snake venom derived from *Naja melanoleuca.*

As shown in Figs. 10a to 10c, among CD4 positive, CD25 positive and Foxp3 positive cells known as markers of regulatory T cells, it was confirmed that the frequency of CD25 positive and Foxp3 positive cells was increased by oral administration of snake venom sample derived from *Naja melanoleuca.*

Therefore, since oral administration of snake venom sample derived from *Naja melanoleuca* individually increased the frequency of CD25-positive and Foxp3-positive cells, which are the main characteristics of regulatory T cells, from this, it was analyzed that oral administration of snake venom sample derived from *Naja melanoleuca* would increase the level of regulatory T cells.

### Example 4-4: Change in regulatory T cells in normal mice orally administered with snake venom derived from Naja melanoleuca

Since results of Example 4-3 above provided a clue that oral administration of snake venom sample derived from *Naja melanoleuca* would increase the level of regulatory T cells, in practice, it was to confirm whether oral administration of snake venom sample derived from *Naja melanoleuca* could increase the level of regulatory T cells.

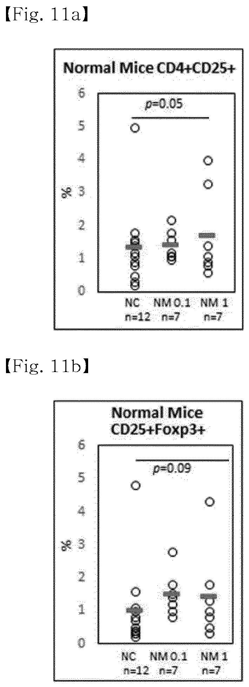

For this, 0.1 µg or 1 µg of snake venom sample derived from *Naja melanoleuca* was orally administered to normal mice, and the levels of cells comprising CD4 positive, CD25 positive and Foxp3 positive in combination from these were confirmed through flow cytometry analysis (Figs. 11a to 11d). At this time, as a control group, mice orally administered with PBS were used.

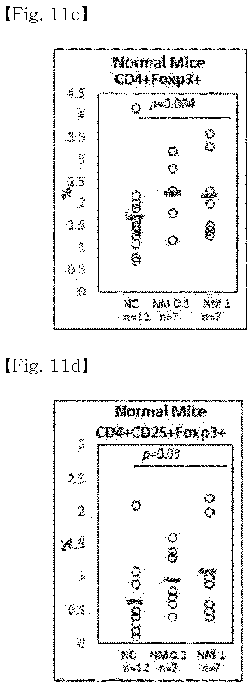

Fig. 11a is a graph showing the results of measuring the changes in CD4 and CD25 positive cells according to the dose in normal mice orally administered with snake venom derived from *Naja melanoleuca,* Fig. 11b is a graph showing the results of measuring the changes in CD25 and Foxp3 positive cells according to the dose in normal mice orally administered with snake venom derived from *Naja melanoleuca,* Fig. 11c is a graph showing the results of measuring the changes in CD4 and Foxp3 positive cells according to the dose in normal mice orally administered with snake venom derived from *Naja melanoleuca,* and Fig. 11d is a graph showing the results of measuring the changes in CD4, CD25 and Foxp3 positive regulatory T cells according to the dose in normal mice orally administered with snake venom derived from *Naja melanoleuca.*

As shown in Figs. 11a to 11d, it was confirmed that snake venom sample derived from *Naja melanoleuca* increased all of the level of each combination of CD4 positive, CD25 positive and Foxp3 positive, known as the main characteristics of regulatory T cells.

Therefore, it was found that oral administration of snake venom sample derived from *Naja melanoleuca* can increase the level of regulatory T cells and, due to the increase in the level of these regulatory T cells, can improve the inflammatory symptoms of rheumatoid arthritis.

### Example 5: Effects of snake venom derived from Agkistrodon piscivorus piscivorus or Naja melanoleuca on metabolite levels in normal mice

### Example 5-1: Level of CRP(C-reactive protein) in plasma

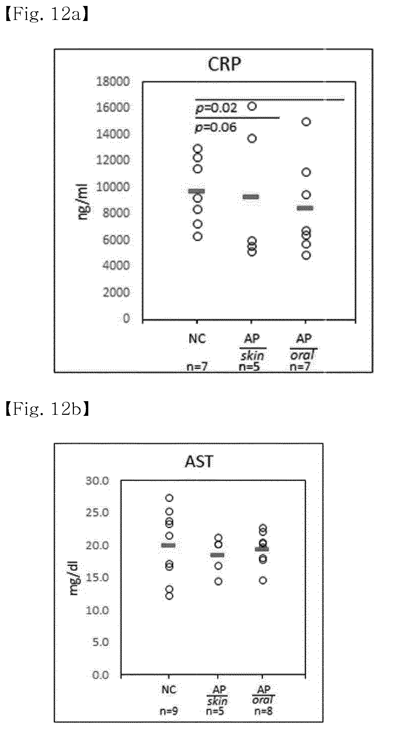

After oral administration or skin application of snake venom sample derived from *Agkistrodon piscivorus piscivorus* to normal mice, plasma from each mouse was collected, and then levels of CRP (C-reactive protein) in plasma, an inflammatory marker, were compared (Fig. 12a). At this time, as a control group, mice orally administered or skin-applied with PBS were used.

Fig. 12a is a graph showing the results of comparing levels of CRP (C-reactive protein) in plasma in normal mice orally administered or skin-applied with snake venom derived from *Agkistrodon piscivorus piscivorus.*

As shown in Fig. 12a, when snake venom derived from *Agkistrodon piscivorus piscivorus* was orally administered or applied on skin, all it was confirmed that the level of CRP in plasma were lowered.

As such, since it was confirmed that the level of CRP in plasma, an inflammatory marker, was decreased by snake venom derived from *Agkistrodon piscivorus piscivorus,* it was found that snake venom derived from *Agkistrodon piscivorus piscivorus* can improve the inflammatory symptoms of rheumatoid arthritis.

### Example 5-2: Level of AST(aspartate aminotransferase) in plasma

After oral administration or skin application of snake venom sample derived from *Agkistrodon piscivorus piscivorus* to normal mice, plasma from each mouse was collected, and then levels of AST(aspartate aminotransferase) in plasma were compared (Fig. 12b). At this time, as a control group, mice orally administered or skin-applied with PBS were used.

Fig. 12b is a graph showing the results of comparing levels of AST(aspartate aminotransferase) in plasma in normal mice orally administered or skin-applied with snake venom derived from *Agkistrodon piscivorus piscivorus.*

As shown in Fig. 12b, when snake venom derived from *Agkistrodon piscivorus piscivorus* was orally administered or applied on skin, all it was confirmed that the level of AST in plasma were lowered.

As shown in FIG. 12b, when the snake venom derived from *Agkistrodon piscivorus piscivorus* was orally administered, there was no significant difference from the control group, but when applied on skin, the level of AST in plasma tended to slightly decrease.

As such, since the level of AST in plasma did not change even when snake venom derived from *Agkistrodon piscivorus piscivorus* was orally administered, it was analyzed that any particular toxicity in the body was not exhibited even when snake venom derived from *Agkistrodon piscivorus piscivorus* was orally administered.

### Example 5-3: Level of ALT(alanine transaminase) in plasma

After oral administration or skin application of snake venom sample derived from *Agkistrodon piscivorus piscivorus* to normal mice, plasma from each mouse was collected, and then levels of ALT(alanine transaminase) in plasma were compared (Fig. 12c). At this time, as a control group, mice orally administered or skin-applied with PBS were used.

Fig. 12c is a graph showing the results of comparing levels of ALT(alanine transaminase) in plasma in normal mice orally administered or skin-applied with snake venom derived from *Agkistrodon piscivorus piscivorus.*

As shown in FIG. 12c, when snake venom derived from *Agkistrodon piscivorus piscivorus* was orally administered or applied on skin, all it was confirmed that the level of ALT in plasma was lowered compared to the control group. In particular, it was confirmed that the level of ALT in plasma exhibited significantly lower value in the case of oral administration than in the case of skin application.

As such, since levels of ALT in plasma tended to be rather decreased when snake venom derived from *Agkistrodon piscivorus piscivorus* was orally administered, it was confirmed once again that any particular toxicity in the body was not exhibited even when snake venom derived from *Agkistrodon piscivorus piscivorus* was orally administered.

### Example 5-4: Level of CRP(C-reactive protein) in plasma

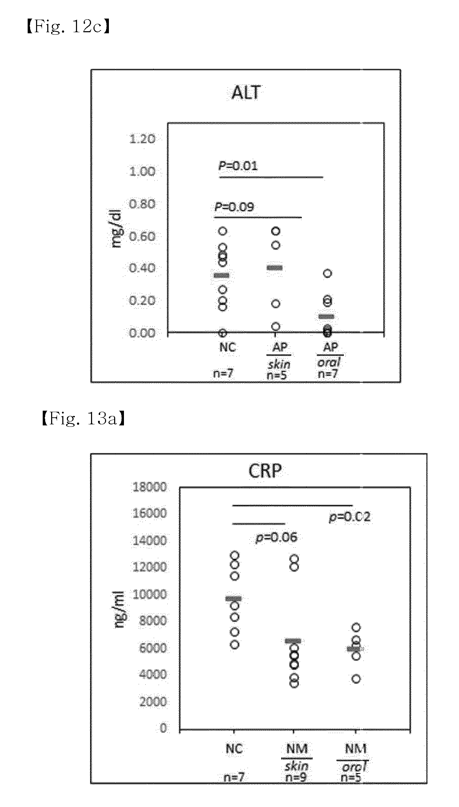

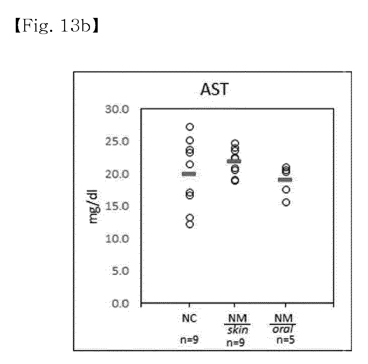

After oral administration or skin application of snake venom sample derived from *Naja melanoleuca* to normal mice, plasma from each mouse was collected, and then levels of CRP(C-reactive protein) in plasma, an inflammatory marker, were compared (Fig. 13a). At this time, as a control group, mice orally administered or skin-applied with PBS were used.

Fig. 13a is a graph showing the results of comparing levels of CRP (C-reactive protein) in plasma in normal mice orally administered or skin-applied with snake venom derived from *Naja melanoleuca.*

As shown in Fig. 13a, when snake venom derived from *Agkistrodon piscivorus piscivorus* was orally administered or applied on skin, all it was confirmed that the level of CRP in plasma were lowered.

As such, since it was confirmed that the level of CRP in plasma, an inflammatory marker, was decreased by snake venom derived from *Naja melanoleuca,* it was found that snake venom derived from *Naja melanoleuca* can improve the inflammatory symptoms of rheumatoid arthritis.

### Example 5-5: Level of AST(aspartate aminotransferase) in plasma

After oral administration or skin application of snake venom sample derived from *Naja melanoleuca* to normal mice, plasma from each mouse was collected, and then levels of AST(aspartate aminotransferase) in plasma were compared (Fig. 13b). At this time, as a control group, mice orally administered or skin-applied with PBS were used.

Fig. 13b is a graph showing the results of levels of comparing AST(aspartate aminotransferase) in plasma in normal mice orally administered or skin-applied with snake venom derived from *Naja melanoleuca.*

As shown in FIG. 13b, when the snake venom derived from *Naja melanoleuca* was orally administered, there was no significant difference from the control group, but when applied on skin, the level of AST in plasma tended to slightly increase.

As such, since the level of AST in plasma did not change even when snake venom derived from *Naja melanoleuca* was orally administered, it was analyzed that any particular toxicity in the body was not exhibited even when snake venom derived from *Naja melanoleuca* was orally administered.

### Example 5-6: Level of ALT(alanine transaminase) in plasma

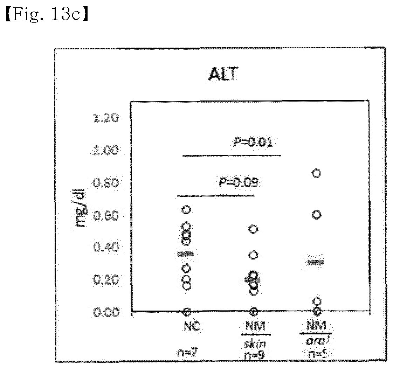

After oral administration or skin application of snake venom sample derived from *Naja melanoleuca* to normal mice, plasma from each mouse was collected, and then levels of ALT(alanine transaminase) in plasma were compared (Fig. 13c). At this time, as a control group, mice orally administered or skin-applied with PBS were used.

Fig. 13c is a graph showing the results of comparing ALT(alanine transaminase) levels in plasma in normal mice orally administered or skin-applied with snake venom derived from *Naja melanoleuca.*

As shown in FIG. 13c, when snake venom derived from *Naja melanoleuca* was orally administered or applied on skin, all it was confirmed that the level of ALT in plasma was lowered compared to the control group. In particular, it was confirmed that the level of ALT in plasma exhibited relatively lower value in the case of skin administration than in the case of oral application.

As such, since levels of ALT in plasma tended to be rather decreased when snake venom derived from *Naja melanoleuca* was orally administered, it was confirmed once again that any particular toxicity in the body was not exhibited even when snake venom derived from *Naja melanoleuca* was orally administered.

### Example 6: Cytokine level analysis

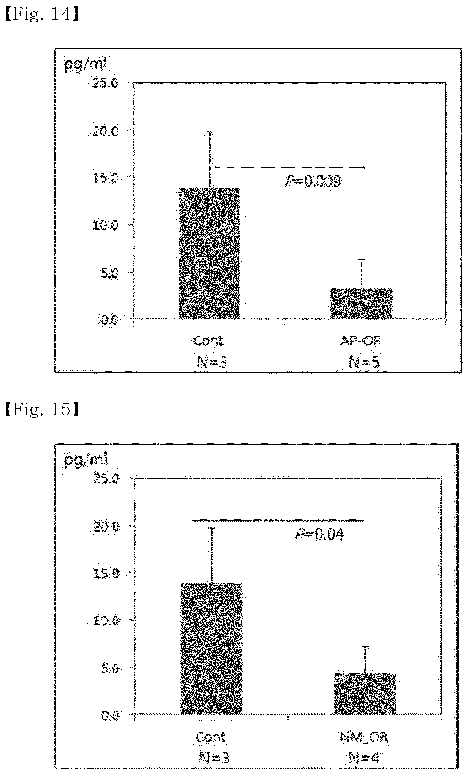

After oral administration of snake venom sample derived from *Agkistrodon piscivorus piscivorus* or *Naja melanoleuca* to normal mice, plasma from mice was collected, and then levels of IL-17 in plasma were compared (Figs. 14 and 15). At this time, as a control group, mice orally administered or skin-applied with PBS were used.

Fig. 14 is a graph showing the results of comparing the level of IL-17 in plasma in normal mice orally administered with snake venom derived from *Agkistrodon piscivorus piscivorus.*

Fig. 15 is a graph showing the results of comparing the level of IL-17 in plasma in normal mice orally administered with snake venom derived from *Naja melanoleuca.*

As shown in Figs. 14 and 15, when snake venom derived from *Agkistrodon piscivorus piscivorus* or *Naja melanoleuca* was orally administered, it was confirmed that the plasma IL-17 level was lowered compared to the control group.

Since said IL-17 is known as an inflammation-inducing cytokine, the result that snake venom derived from *Agkistrodon piscivorus piscivorus* or *Naja melanoleuca* lowers the IL-17 level, that is, was analyzed to mean that snake venom derived from *Agkistrodon piscivorus piscivorus* or *Naja melanoleuca* could improve the inflammatory symptoms of rheumatoid arthritis.

### Example 7: Histological analysis of arthritis model mice

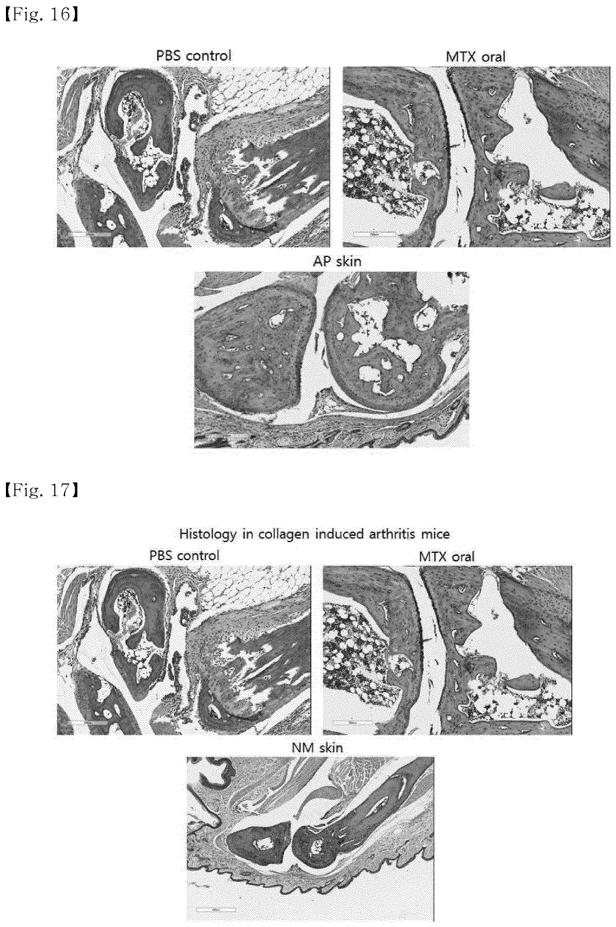

To the arthritis model mice produced in Example 1 above, MTX was orally administered or snake venom derived from *Agkistrodon piscivorus piscivorus* or *Naja melanoleuca* was applied on skin, and after collecting the arthritis area tissue from mice, a tissue slice thereof was obtained, and was confirmed under a microscope to confirm whether inflammatory cells in the tissue are present (Figs. 16 and 17). At this time, as a control group, PBS orally administered mice were used. In addition, the tissue slices were prepared by decalcifying the collected tissue in a decalcification solution, fixing it to make a paraffin block, cutting it to obtain a slice, and then performing H&E staining.

Fig. 16 is a microphotograph showing the joint tissue of arthritis model mice orally administered with MTX or applied on skin with snake venom derived from *Agkistrodon piscivorus piscivorus.*

Fig. 17 is a microphotograph showing the joint tissue of arthritis model mice orally administered with MTX or applied on skin with snake venom derived from *Naja melanoleuca.*

As shown in Figs. 16 and 17, it was confirmed that inflammatory cells were present at the joint area in the tissue of the control group, but it was confirmed that no inflammatory cell was present at the joint area in the tissue of the arthritis model mice orally administered with MTX or applied on skin with snake venom derived from *Agkistrodon piscivorus piscivorus* or *Naja melanoleuca.*

Therefore, it was found that snake venom derived from *Agkistrodon piscivorus piscivorus* or *Naja melanoleuca* could improve the inflammatory symptoms of rheumatoid arthritis.

## Claims

1. A pharmaceutical composition for use in preventing or treating rheumatoid arthritis, comprising snake venom derived from *Naja melanoleuca.*

2. The pharmaceutical composition for use in preventing or treating rheumatoid arthritis according to Claim 1,
wherein the composition further comprises a pharmaceutically acceptable carrier, an excipient or a diluent.

3. A food composition for use in preventing or improving rheumatoid arthritis, comprising snake venom derived from *Naja melanoleuca.*

## Patentansprüche

1. Pharmazeutische Zusammensetzung zur Verwendung bei der Vorbeugung oder Behandlung von rheumatoider Arthritis, umfassend Schlangengift, das aus *Naja melanoleuca* gewonnen ist.

2. Pharmazeutische Zusammensetzung zur Verwendung bei der Vorbeugung oder Behandlung von rheumatoider Arthritis nach Anspruch 1,
wobei die Zusammensetzung ferner einen pharmazeutisch akzeptablen Träger, einen Hilfsstoff oder ein Verdünnungsmittel umfasst.

3. Nahrungsmittelzusammensetzung zur Verwendung bei der Vorbeugung oder Linderung von rheumatoider Arthritis, umfassend Schlangengift, das aus *Naja melanoleuca* gewonnen ist.

## Revendications

1. Composition pharmaceutique pour utilisation dans la prévention ou le traitement de la polyarthrite rhumatoïde, comprenant du venin de serpent dérivé de *Naja melanoleuca.*

2. Composition pharmaceutique pour utilisation dans la prévention ou le traitement de la polyarthrite rhumatoïde selon la revendication 1,
dans laquelle la composition comprend en outre un support pharmaceutiquement acceptable, un excipient ou un diluant.

3. Composition alimentaire pour utilisation dans la prévention ou l'amélioration de la polyarthrite rhumatoïde, comprenant du venin de serpent dérivé de *Naja melanoleuca.*
